# EUROPÄISCHE PATENTSCHRIFT

(11) **EP 1 415 638 B1**
(45) Veröffentlichungstag und Bekanntmachung des Hinweises auf die Patenterteilung: **29.11.2006**
(21) Anmeldenummer: 02024103.0
(22) Anmeldetag: 29.10.2002
(51) Int. Cl.: A61K 8/11, A61K 8/97, B01J 13/02

(54) **Wässrige Wirkstoffzubereitungen enthaltend pflanzenextrakte und mikroverkapselte fettlösliche Wirkstoffe**
Aqueous active substance preparations containing plant extracts and liposoluble microencapsulated active agents
Préparations aqueuses de principe actif contenants des extraits végétaux et des agents actifs liposolubles microencapsulés

(43) Veröffentlichungstag der Anmeldung: 06.05.2004
(73) Patentinhaber: Cognis IP Management GmbH, 40589 Düsseldorf (DE)
(72) Erfinder: De Moragas, Maria, Dr., 08310 Argentona (Barcelona) (ES); Viladot Petit, Josep-Lluis, Dr., 08018 Barcelona (ES)

(56) Entgegenhaltungen:
- EP-A- 1 243 247
- EP-A- 1 243 318
- EP-A- 1 243 320
- EP-A- 1 243 325
- EP-A- 1 243 326
- DE-A- 10 117 842
- "Conference Proceedings" CONFERENCE INTEX, SHANGHAI, CHINA, 19-21 MARCH 2002, [Online] XP002234276 Gefunden im Internet: <URL:HTTP://WWW.STEPEX.COM/CONFERENCE_PROC EEDINGS/CONFERENCE_PROCEEDINGS/PCIA_MARCH2 002CONTENTS.HTML> [gefunden am 2003-03-11]
- MAEYAMA L (MEAYAMA L=COGNIS JAPAN, CARE CHEMICALS ASIA PACIFIC): "PERFECT ANTI-AGING: ENCAPSULATED STABILIZED RETINOL FOR ANTI-WRINKLE AND PISUM SATIVUM EXTRACT FOR FIRMING" PERSONAL CARE, 2002, 3, 3, 17-22, XP001145883
- "Active Ingredients Glossary" JANSSEN COSMECEUTICAL CARE, [Online] XP002234277 Gefunden im Internet: <URL:HTTP://WWW.JANSSENBEAUTY.COM/US/GLOSS ARY.HTM> [gefunden am 2003-03-11]

## Beschreibung

Die Erfindung befindet sich auf dem Gebiet der mikroverkapselten Wirkstoffe und betrifft die Verwendung von Zubereitungen, welche die mikroverkapselten Wirkstoffe zusammen mit Pflanzenextrakten enthalten in der Textil- und Papiertechnik.

### Stand der Technik

Von modernen Verbrauchern wird immer häufiger der Anspruch erhoben, dass die Dinge des täglichen Lebens über ihre direkte Funktionsweise hinaus Vorteile aufweisen. Dass kosmetische Zubereitungen nicht nur einen pflegenden Charakter besitzen, sondern beispielsweise Haut und Haare auch vor schädigenden Umwelteinflüssen schützen bzw. bereits bestehende Läsuren beheben sollen, ist heutzutage Standard. Inzwischen halten kosmetische Wirkstoffe jedoch auch Einzug in den Bereich der Textiltechnik : Damenstrümpfe werden mit verkapselten Wirkstoffen ausgerüstet, die während des Tragens verzögert freigesetzt werden und den vom Stehen ermüdeten Beinen beispielsweise ein anhaltendes Frischegefühl verleihen sollen. Ein anderes Beispiel sind Windeln oder Binden, die an der Oberfläche oder in der Flüssigkeit speichernden Schicht ebensolche Wirkstoffe enthalten, die empfindliche Haut vor Reizungen schützen oder die Geruchsbildung vermeiden sollen. Schließlich werden auch Papiere mit Wirkstoffzubereitungen ausgerüstet, die ihnen einen angenehmen Griff oder Geruch verleihen.

Als kosmetische Wirkstoffe für diese sehr unterschiedlichen Anwendungsgebiete kommen beispielsweise Retinol, Tocopherol sowie deren Derivate aber auch Polyphenole, Flavonoide und dergleichen in Frage. Bei der Formulierung derartiger Zubereitungen bestehen nun zwei Probleme: während Retinole und Tocopherole lipophil sind, besitzen Polyphenole und Flavonoide eine genau entgegengesetzte Polarität, so dass eine gemeinsame Verwendung, zumal ohne Verwendung von Hilfsstoffen wie Emulgatoren, Solubilisatoren und dergleichen, schwierig ist. Zum anderen sind die genannten fettlöslichen Wirkstoffe häufig instabil; so wird Retinol beispielsweise unter dem Einfluss von Licht sehr rasch abgebaut und damit unwirksam.

Die Aufgabe der vorliegenden Erfindung hat somit darin bestanden, Zubereitungen zur Verfügung zu stellen, die nebeneinander lipophile Wirkstoffe wie z.B. das wenig chemisch beständige Retinol und hydrophile Pflanzenextrakte in einer stabilisierten Form enthalten. Insbesondere galt es sowohl das Problem der durch die gegensätzlichen Polaritäten begründeten mangelnden Mischbarkeit zu lösen, als auch die fettlöslichen Wirkstoffe gegen (photo-)chemischen Abbau zu stabilisieren. Eine weitere Aufgabe hat schließlich darin bestanden, solche Zubereitungsformen zu entwickeln, die sich zur Ausrüstung von textilen Flächengebilden oder Papier.

### Beschreibung der Erfindung

Gegenstand der Erfindung ist die Verwendung von wässrige Wirkstoffzubereitungen, enthaltend Pflanzenextrakte und mikroverkapselte fettlösliche Wirkstoffe zur Ausrüstung von Textilen Flächengebilden oder non-wovens.

Überraschenderweise wurde gefunden, dass diese Zubereitungen zur Ausrüstung von Textilien Papier dient. Insbesondere erlauben die Zubereitungen die gemeinsame Verwendung von (photo-)chemisch instabilen lipophilen Wirkstoffen, wie beispielsweise Retinol, und hydrophilen Wirkstoffen vom Typ der Pflanzenextrakte. Die Verkapselung der fettlöslichen Wirkstoffe bewirkt dabei ihre Stabilisierung gegenüber einem unerwünschten chemischen Abbau, während das Problem der fehlenden Mischbarkeit beider Wirkstofftypen dadurch gelöst wird, dass man die Mikrokapseln in einer wässrigen, die hydrophilen Wirkstoffe enthaltenden Phase gegebenenfalls unter Mitverwendung von geeigneten polymeren Verdickungsmitteln dispergiert.

### Pflanzliche Wirkstoffe

Als pflanzliche Wirkstoffe kommen beispielsweise die Extrakte der folgenden Pflanzen bzw. deren aktive Prinzipien in Frage:
- *Aloe Vera*
- *Arcticum lappa*
- *Argania spinosa*
- *Arrabidea chica*
- *Baptista tinctoria,*
- *Cajanus cajun*
- *Camellia sinensis* (Grüner Tee)
- *Cassia alata*
- *Craterostigma plantigineum*
- *Ginkgo biloba* (Ginkgo)
- *Glycyrrhiza glabra*
- *Grifola frondosa*
- *Hibiscus asculentus* (Hibiscus)
- *Myrothamnus flabellifolia*
- *Olea europensis* (Olive)
- *Prunus dulcis* (Süssmandel)
- *Pterocarpus marsupium*
- *Salix alba* (Weide)
- *Trifolium pratense* (Rotklee)
- *Vacinium myrtillus* (Blaubeere)
- *Vigna acontifolia*
- *Vitis vinifera* (Wein)
- *Walteria indica*

Die Herstellung der Extrakte kann in an sich bekannter Weise erfolgen, d.h. beispielsweise durch wässrigen, alkoholischen oder wässrig-alkoholischen Auszug der Pflanzen bzw. Pflanzenteile bzw. der Blätter oder Früchte. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Blätter liegen im Bereich von 1 bis 25, insbesondere 2 bis 22 Gew.-%. Die vorliegenden Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 25 Gew.-% aufweisen, können als solche eingesetzt werden, es ist jedoch ebenfalls möglich, das Lösungsmittel durch Trocknung, insbesondere durch Sprüh- oder Gefriertrocknung vollständig zu entfernen, wobei ein intensiv rot gefärbter Feststoff zurückbleibt. Die Extrakte können auch als Ausgangsstoffe für die Gewinnung der oben genannten reinen Wirkstoffe dienen, sofern diese nicht auf synthetischem Wege einfacher und kostengünstiger hergestellt werden können. Demzufolge kann der Wirkstoffgehalt in den Extrakten 5 bis 100, vorzugsweise 50 bis 98 Gew.-% betragen. Die Extrakte selbst können als wässrige und/oder in organischen Solventien gelöste Zubereitungen sowie als sprüh- bzw. gefriergetrocknete, wasserfreie Feststoffe vorliegen. Als organische, gegebenenfalls mit Wasser mischbare Lösungsmittel kommen in diesem Zusammenhang beispielsweise die aliphatischen Alkohole mit 1 bis 6 Kohlenstoffatomen (z.B. Ethanol), Ketone (z.B. Aceton), Halogenkohlenwasserstoffe (z.B. Chloroform oder Methylenchlorid), niedere Ester oder Polyole (z.B. Glycerin oder Glycole) in Frage.

### Fettlösliche Wirkstoffe

Als fettlösliche Wirkstoffe, die vorzugsweise bei 20 °C in flüssiger Form vorliegen, kommen beispielsweise Retinolsäure, Bisabolol, Panthenol, die diversen Tocopherole und Carotinoide sowie deren Gemische in Frage. Bevorzugt ist der Einsatz von Retinol, da dieser Wirkstoff häufige Anwendung im Bereich der Kosmetik findet, jedoch besonders leicht photochemisch abgebaut wird.

### Mikrokapseln

Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide). Chitosanmikrokapseln und Verfahren zu ihrer Herstellung sind Gegenstand früherer Patenanmeldungen der Patentanmelderin **[WO 01/01926, WO 01/01927, WO 01/01928, WO 01/01929].** Mikro- und Nanokapseln sind auch aus EP-A-1243318, EP-A-1243320, EP-A-1243326 bekannt.

In bevorzugten Ausführungsformen der vorliegenden Erfindung werden Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, eingesetzt, die beispielsweise erhalten werden, indem man
(a1) aus Gelbildnern, kationischen Polymeren (z.B. Chitosan) und Wirkstoffen eine Matrix zubereitet,
(a2) gegebenenfalls die Matrix in einer Ölphase dispergiert,
(a3) die dispergierte Matrix mit einer wässrigen Lösung eines Anionpolymers behandelt und gegebenenfalls dabei die Ölphase entfernt.
oder
(b1) aus Gelbildnern, anionischen Polymeren und Wirkstoffen eine Matrix zubereitet,
(b2) gegebenenfalls die Matrix in einer Ölphase dispergiert,
(b3) die dispergierte Matrix mit einer wässrigen Lösung eines Kationpolymers behandelt und gegebenenfalls dabei die Ölphase entfernt.
oder
(c1) fettlösliche Wirkstoffe mit Schmelzpunkten unterhalb von 20 °C und Wachse mit Schmelzpunkten oberhalb von 20 °C bei einer Temperatur oberhalb des Schmelzpunktes der Wachse zu einer Ölphase verarbeitet,
(c2) die so erhaltene Ölphase mit einer wässrigen Lösung eines Anionpolymers vermischt, und
(c3) die so erhaltene O/W-Emulsion auf eine Temperatur unterlab des Schmelzpunktes der Wachse abkühlt und mit der wässrigen Lösung eines Kationpolymers in Kontakt bringt,
oder
(d1) fettlösliche Wirkstoffe mit Schmelzpunkten unterhalb von 20 °C und Wachse mit Schmelzpunkten oberhalb von 20 °C bei einer Temperatur oberhalb des Schmelzpunktes der Wachse zu einer Ölphase verarbeitet,
(d2) die so erhaltene Ölphase mit einer wässrigen Lösung eines Kationpolymers vermischt, und
(d3) die so erhaltene O/W-Emulsion auf eine Temperatur unterhalb des Schmelzpunktes der Wachse abkühlt und mit der wässrigen Lösung eines Anionpolymers in Kontakt bringt.

### Herstellverfahren Mikrokapseln

Zur Herstellung der mikroverkapselten Wirkstoffe stellt man beispielsweise eine 1 bis 10, vorzugsweise 2 bis 5 Gew.-%ige wässrige Lösung eines Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche ein kationisches Polymer, vorzugsweise Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Kationpolymer und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des kationischen Polymers in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Kationpolymeren durchführen.

In einem alternativen Verfahren erfolgt die Herstellung der Mikrokapseln in mehreren Schritten. Zunächst wird eine Ölphase hergestellt, indem man ein Wachs über seinen Schmelzpunkt auf beispielsweise 60 bis 80 °C erwärmt und den bei Raumtemperatur flüssigen fettlöslichen Wirkstoff darin löst. Üblicherweise werden dabei Wirkstoffe und Wachse im Gewichtsverhältnis 3 : 7 bis 0,5 : 9,5 und vorzugsweise 2 : 8 bis 1 : 9 eingesetzt. Parallel wird eine wässrige Phase vorbereitet, welche neben einem anionischen Polymer auch noch - falls gewünscht - einen Gelbildner und/oder Emulgator enthalten kann. Üblicherweise enthält die wässrige Phase 1 bis 5 Gew.-% Polymer sowie jeweils 1 bis 2 Gew.-% der beiden anderen Komponenten. Die beiden Phasen werden als dann in der Wärme unter intensivem Rühren miteinander vermischt, wobei eine W/O-Emulsion entsteht. Zur Ausbildung der Hüllmembran wird die Emulsion zunächst auf eine Temperatur von 40 bis 50 °C abgekühlt und dann portionsweise mit einer 1 bis 5 Gew.-%igen wässrigen Lösung eines Kationpolymers versetzt. Anion- und Kationpolymer sind dabei in den Verfahrensschritten austauschbar, d.h. die Emulsionsbildung kann auch unter Einsatz des kationischen Polymers erfolgen, während die Hülle dann durch Fällung mit dem Anionpolymer erzeugt wird. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert gewöhnlich auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diesten von Fettsäuren, Polyacrylaten, Polyacrylamiden und dergleichen noch unterstützt werden kann. Abschließend können die Mikrokapseln - falls gewünscht - von der wässrigen Phase beispielsweise durch Dekantieren, Filtrieren oder Zentrifugieren abgetrennt werden. Auf diesem Wege werden Mikrokapseln erhalten, welche im Mittel einen Durchmesser im Bereich von vorzugsweise etwa 0,1 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig.

### Verdickungsmittel

In einer bevorzugten Ausführungsform der vorliegenden Erfindung enthalten die Zubereitungen als weitere Komponente (c) Verdickungsmittel. Diese besitzen die Aufgabe, den wässrigen Zubereitungen eine solch hohe Viskosität zu verleihen, dass die Mikrokapseln stabil dispergiert bleiben, d.h. nicht im Laufe der Lagerung zu boden sinken. Unter dem Begriff erhöhter Viskosität ist somit eine solche Rheologie zu verstehen, die die Stabilisierung der Mikrokapseln sicherstellt. Üblicherweise liegen derartige Viskositäten (bestimmt nach Brookfield, RVT-Viskosimeter, 20 °C, Spindel 1, 10 Upm) oberhalb von 100 und vorzugsweise oberhalb von 500 mPas, vorzugsweise im Bereich von 200 bis 2.000 und insbesondere 500 bis 1.000 mPas.

Geeignete Verdickungsmittel sind alle die Stoffe, die den Zubereitungen eine entsprechend hohe Viskosität verleihen. Vorzugsweise handelt es sich jedoch um polymere Verbindungen, da diese in der Lage sind, in den wässrigen Zubereitungen ein dreidimensionales Netz aufzubauen, in welchem die Mikrokapseln stabilisiert werden. Typische Beispiele sind Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt.

### Wässrige Zubereitungen

In einer bevorzugten Ausführungsform der vorliegenden Erfindung enthalten die wässrigen Zubereitungen
(a) 0,01 bis 5, vorzugsweise 0,1 bis 3 und insbesondere 0,5 bis 1 Gew.-% Pflanzenextrakte,
(b) 0,1 bis 10, vorzugsweise 0,5 bis 8 und insbesondere 1 bis 3 Gew.-% mikroverkapselte fettlösliche Wirkstoffe, und
(c) 0 bis 5, vorzugsweise 0,1 bis 3 und insbesondere 0,5 bis 2 Gew.-% Verdickungsmittel
mit der Maßgabe, dass sich die Mengenangaben mit Wasser zu 100 Gew.-% ergänzen. Eine besonders bevorzugte Ausführungsform der Erfindung besteht in Zubereitungen, die Aloe Vera zusammen mit mikroverkapseltem Retinol enthalten.

### Gewerbliche Anwendbarkeit

Weitere Gegenstände der vorliegenden Erfindung betreffen die Verwendung der neuen Zubereitungen, nämlich
- zur Ausrüstung von textilen Flächengebilden, wie Garnen, Geweben und Textilien,
- zur Ausrüstung von sogenannten "non-wovens", insbesondere Windeln, Binden und dergleichen sowie
- zur Ausrüstung von Papieren,
wobei die Einsatzmenge (entsprechend der Menge an nicht-wässrigen Wirkstoffen, die nach der Behandlung auf dem Substrat verbleibt) der Zubereitungen bezogen auf das Substrat gewöhnlich 0,01 bis 5, vorzugsweise 0,1 bis 3 und insbesondere 0,5 bis 2 Gew.-% beträgt.

### Beispiele

### Referenz-Beispiel 1

### Herstellung einer Chitosangesichtsmaske mit Aloe Vera und Retinol

Eine Suspension aus 2 kg Chitosan (Hydagen ^{®}CMFP, Cognis), 98 kg Wasser und 0,346 kg L-(+)-Milchsäure wurden mittels einer Kolloidmühle solange bei einer Temperatur von 40 °C homogenisiert, bis eine Viskosität von 23000 mPas erreicht wurde. Danach wurde die Suspension auf 10 °C abgekühlt und im Vakuum entgast. Jeweils 9 kg der Suspension wurden mit 360 g einer wässrigen Lösung von Natriumhydrogencarbonat (= 8,05 Gew.-%ige wässerige Natriumhydrogencarbonatlösung) sowie 1 kg einer wässrigen Zubereitung enthaltend 2 Gew.-% Aloe Vera Extrakt und 1 Gew.-% mikroverkapseltes Retinol (Retinol Primaspheres®, Cognis Iberia) für 2 Minuten gemischt und anschließend in Formen gegossen. Die Schichtdicke der Suspension in der Form betrug 22 mm. Nach einer Ruhezeit von 3h wurde die Suspension eingefroren und anschließend die gefrorenen Platten bei 80°C und 1 mbar gefriergetrocknet. Die getrockneten Blöcke wurden anschließend auf die gewünschte Größe und Dicke geschnitten. (Dicke: 1,2 mm, Größe: 20 x 30 cm).

### Referenz-Beispiel 2

### Herstellung einer Chitosangesichtsmaske mit Grünem Tee und Retinol

Eine Suspension aus 2 kg Chitosan, 98 kg Wasser, 0,292 kg Glykolsäure, 0,1 kg Cellulosefasern, 1 kg einer wässrigen Zubereitung enthaltend 2 Gew.-% Grünem Tee Extrakt und 1 Gew.-% mikroverkapseltes Retinol (Retinol Primaspheres^{®}, Cognis Iberia) sowie 0,08 kg Emulgator PEG-30 Glyceryl Stearat (Tagat S^{®}, Tego Cosmetics, Goldschmidt) wurden mittels einer Kolloidmühle solange bei Temperaturen von 50°C homogenisiert, bis eine Viskosität von 26000 mPas erreicht wurde. Danach wurde die Suspension auf 10 °C abgekühlt und im Vakuum entgast. Jeweils 9 kg der Suspension wurden mit 360 g einer wässerigen Lösung von Natriumhydrogencarbonat (= 8,05 Gew.-%ige wässrige Natriumhydrogencarbonatlösung) für 1 Minute gemischt und anschließend in Formen gegossen. Nach einer Ruhezeit von 30 min wurde die Suspension eingefroren und die gefrorenen Platten anschließend bei 80°C und 1 mbar gefriergetrocknet. Die getrockneten Blöcke wurden anschließend auf die gewünschte Größe und Dicke geschnitten (Dicke: 1,5 mm, Größe 20 x 30 cm).

## Patentansprüche

1. Verwendung von wässrige Wirkstoffzubereitungen, enthaltend
(a) Pflanzenextrakte und
(b) mikroverkapselte fettlösliche Wirkstoffe.
mit der Maßgabe, dass die Extrakte von Pflanzen ausgewählt sind aus der Gruppe, die gebildet wird von *Aloe Vera, Arcticum lappa, Argania spinosa, Arrabidea chica, Baptista tinctoria, Cajanus cajun, Camellia sinensis, Cassia alata, Craterostigma plantigineu, Ginkgo biloba, Glycyrrhiza glabra, Grifola frondos, Hibiscus asculentus, Myrothamnus flabellifolia, Olea europensis, Prunus dulcis, Pterocarpus marsupium, Salix alba, Trifolium pratense, Vacinium myrtillus, Vigna acontifolia, Vitis vinifera, Walteria indica* sowie deren Gemischen zur Ausrüstung von textilen Flächengebilden oder non-wovens.

2. Verwendung nach den Anspruch 1, **dadurch gekennzeichnet, dass** die Zubereitungen fettlösliche Wirkstoffe enthalten, die ausgewählt sind aus der Gruppe, die gebildet wird von Retinol, Retinolsäure, Bisabolol, Panthenol, Tocopherolen, Carotinoiden und deren Gemischen.

3. Verwendung nach einem Anspruch 1 oder 2, **dadurch gekennzeichnet, dass** die Zubereitungen Mikrokapseln mit mittleren Durchmessern im Bereich von 0,0001 bis 5 mm, bestehend aus einer Hüllmembran und einer die Wirkstoffe enthaltenden Matrix enthalten, welche hergestellt werden, indem man
(a1) aus Gelbildnern, Chitosanen und Wirkstoffen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.
oder
(b1) aus Gelbildnern, anionischen Polymeren und Wirkstoffen eine Matrix zubereitet,
(b2) gegebenenfalls die Matrix in einer Ölphase dispergiert,
(b3) die dispergierte Matrix mit wässrigen Chitosanlösungen behandelt und gegebenenfalls dabei die Ölphase entfernt.

4. Verwendung nach mindestens einem der Ansprüche 1 bis 3, **dadurch gekennzeichnet, dass** die Zubereitungen Mikrokapseln mit mittleren Durchmessern im Bereich von 0,0001 bis 5 mm, bestehend aus einer Hüllmembran und einer die Wirkstoffe enthaltenden Matrix enthalten, welche hergestellt werden, indem man
(c1) fettlösliche Wirkstoffe mit Schmelzpunkten unterhalb von 20 °C und Wachse mit Schmelzpunkten oberhalb von 20 °C bei einer Temperatur oberhalb des Schmelzpunktes der Wachse zu einer Ölphase verarbeitet,
(c2) die so erhaltene Ölphase mit einer wässrigen Lösung eines Anionpolymers vermischt, und
(c3) die so erhaltene O/W-Emulsion auf eine Temperatur unterhalb des Schmelzpunktes der Wachse abkühlt und mit der wässrigen Lösung eines Kationpolymers in Kontakt bringt,
oder
(d1) fettlösliche Wirkstoffe mit Schmelzpunkten unterhalb von 20 °C und Wachse mit Schmelzpunkten oberhalb von 20 °C bei einer Temperatur oberhalb des Schmelzpunktes der Wachse zu einer Ölphase verarbeitet,
(d2) die so erhaltene Ölphase mit einer wässrigen Lösung eines Kationpolymers vermischt, und
(d3) die so erhaltene O/W-Emulsion auf eine Temperatur unterlab des Schmelzpunktes der Wachse abkühlt und mit der wässrigen Lösung eines Anionpolymers in Kontakt bringt.

5. Verwendung nach mindestens einem der Ansprüche 1 bis 4, **dadurch gekennzeichnet, dass** die Zubereitungen als weitere Komponente (c) Verdickungsmittel enthalten.

6. Verwendung nach Anspruch 5, **dadurch gekennzeichnet, dass** die Zubereitungen als Komponente (c) Verdickungsmittel enthalten, die ausgewählt sind aus der Gruppe, die gebildet wird von hydrophilen Kieselsäuren, Polysacchariden, Carboxymethylcellulosen, Hydroxyethyl- und Hydroxypropylcellulosen, höhermolekularen Polyethylenglycolmono- und - diestem von Fettsäuren, Polyacrylaten, Polyacrylamiden, Polyvinylalkoholen, Polyvinylpyrrolidonen sowie Bentoniten.

7. Verwendung nach mindestens einem der Ansprüche 1 bis 6, **dadurch gekennzeichnet, dass** die Zubereitungen
(a) 0,01 bis 5 Gew.-% Pflanzenextrakte,
(b) 0,1 bis 10 Gew.-% mikroverkapselte fettlösliche Wirkstoffe, und
(c) 0 bis 5 Gew.-% Verdickungsmittel
mit der Maßgabe enthalten, dass sich die Mengenangaben mit Wasser zu 100 Gew.-% ergänzen.

## Claims

1. Use of aqueous active-component preparations containing
(a) plant extracts and
(b) microencapsulated liposoluble active components,
with the proviso that the extracts of plants are selected from the group consisting of *Aloe vera, Arcticum lappa, Argania spinosa, Arrabidea chica, Baptista tinctoria, Cajanus cajun, Camellia sinensis, Cassia alata, Craterostigma plantigineu, Gingko biloba, Glycyrrhiza glabra, Grifola frondos, Hibiscus asculentus, Myrothamnus flabellifolia, Olea europensis, Prunus dulcis, Pterocarpus marsupium, Salix alba, Trifolium pratense, Vacinium myrtillus, Vigna acontifolia, Vitis vinifera, Waltheria indica* and mixtures thereof,
for finishing flat textile materials or nonwovens.

2. Use as claimed in claim 1, **characterized in that** the preparations contain liposoluble active components selected from the group consisting of retinol, retinolic acid, bisabolol, panthenol, tocopherols, carotinoids and mixtures thereof.

3. Use as claimed in claim 1 or 2, **characterized in that** the preparations contain microcapsules with mean diameters of 0.0001 to 5 mm consisting of a membrane and a matrix containing the active components which are prepared by
(a1) preparing a matrix from gel formers, chitosans and active components,
(a2) optionally dispersing the matrix in an oil phase,
(a3) treating the dispersed matrix with aqueous solutions of anionic polymers and removing the oil phase in the process
or by
(b1) preparing a matrix from gel formers, anionic polymers and active components,
(b2) optionally dispersing the matrix in an oil phase,
(b3) treating the dispersed matrix with aqueous chitosan solutions and optionally removing the oil phase in the process.

4. Use as claimed in at least one of claims 1 to 3, **characterized in that** the preparations contain microcapsules with mean diameters of 0.0001 to 5 mm consisting of a membrane and a matrix containing the active components which are prepared by
(c1) processing liposoluble active components with melting points below 20°C and waxes with melting points above 20°C at a temperature above the melting point of the waxes to form an oil phase,
(c2) mixing the oil phase thus obtained with an aqueous solution of an anionic polymer and
(c3) cooling the o/w emulsion thus obtained to a temperature below the melting point of the waxes and contacting it with an aqueous solution of a cationic polymer
or by
(d1) processing liposoluble active components with melting points below 20°C and waxes with melting points above 20°C at a temperature above the melting point of the waxes to form an oil phase,
(d2) mixing the oil phase thus obtained with an aqueous solution of a cationic polymer and
(d3) cooling the o/w emulsion thus obtained to a temperature below the melting point of the waxes and contacting it with an aqueous solution of an anionic polymer.

5. Use as claimed in at least one of claims 1 to 4, **characterized in that** the preparations contain thickeners as an additional component (c).

6. Use as claimed in claim 5, **characterized in that** the preparations contain as component (c) thickeners selected from the group consisting of hydrophilic silicas, polysaccharides, carboxymethyl celluloses, hydroxyethyl and hydroxypropyl celluloses, relatively high molecular weight polyethylene glycol mono- and diesters of fatty acids, polyacrylates, polyacrylamides, polyvinyl alcohols, polyvinyl pyrrolidones and bentonites.

7. Use as claimed in at least one of claims 1 to 6, **characterized in that** the preparations contain
(a) 0.01 to 5% by weight plant extracts,
(b) 0.1 to 10% by weight microencapsulated liposoluble active components and
(c) 0 to 5% by weight thickeners,
with the proviso that the quantities shown add up to 100% by weight with water.

## Revendications

1. Utilisation de préparations d'agents actifs aqueuses contenant
(a) des extraits végétaux et
(b) des agents actifs solubles dans les graisses, microencapsulés
étant précisé que les extraits végétaux sont choisis dans le groupe constitué *d'Aloe Vera, Arcticum lappa, Argania spinosa, Arrabidea chica, Baptista tinctoria, Cajanus cajun, Carmellia sinensis, Cassia alata, Craterostigma plantigineu, Ginkgo biloba, Glycyrrhiza glabra, Grifola frondos, Hibiscus asculentus, Myrothamnus flabellifolia, Olea europensis, Prunis dulcis, Pterocarpus marsupium, Salix alba, trifolium pratense, Vacinium myrtillus, Vigna acontifolia, Vitis vinifera, Walteria indica* ainsi que leurs mélanges, pour apprêter des articles plats textiles ou des non-tissés.

2. Utilisation selon la revendication 1,
**caractérisée en ce que**
les préparations contiennent des agents actifs solubles dans les graisses choisis dans le groupe formé par le rétinol, l'acide rétinolique, le bisabolol, le panthénol, les tocophérols, les carotinoïdes et leurs mélanges.

3. Utilisation selon l'une des revendications 1 ou 2,
**caractérisée en ce que**
les préparations contiennent des microcapsules ayant des diamètres moyens de l'ordre de 0,0001 à 5 mm, constituées d'une membrane extérieure et d'une matrice contenant les agents actifs, que l'on obtient :
(a1) en préparant une matrice à partir de gélifiants, de chitosanes et d'agents actifs,
(a2) en dispersant le cas échéant la matrice dans une phase huileuse,
(a3) en traitant la matrice dispersée avec des solutions aqueuses de polymères anioniques tout en éliminant, le cas échéant, la phase huileuse,
ou
(b1) en préparant une matrice à partir de gélifiants, de polymères anioniques et d'agents actifs,
(b2) en dispersant le cas échéant la matrice dans une phase huileuse,
(b3) en traitant la matrice dispersée avec des solutions aqueuses de chitosanes tout en éliminant, le cas échéant, la phase huileuse.

4. Utilisation selon au moins l'une des revendications 1 à 3,
**caractérisée en ce que**
les préparations contiennent des microcapsules ayant des diamètres moyens de l'ordre de 0,0001 à 5 mm, constituées d'une membrane extérieure et d'une matrice contenant les agents actifs que l'on obtient :
(c1) en mettant en oeuvre de façon à obtenir une phase huileuse, des agents actifs solubles dans les graisses ayant des points de fusion inférieurs à 20 °C et des cires ayant des points de fusion supérieurs à 20 °C à une température supérieure au point de fusion des cires,
(c2) en mélangeant la phase huileuse ainsi obtenue à une solution aqueuse d'un polymère anionique et
(c3) en refroidissant l'émulsion H/E ainsi obtenue à une température inférieure au point de fusion des cires et en la mettant en contact avec la solution aqueuse d'un polymère cationique
ou
(d1) en mettant en oeuvre de façon à obtenir une phase huileuse des agents actifs solubles dans les graisses ayant des points de fusion inférieurs à 20 °C et des cires ayant des points de fusion supérieurs à 20 °C à une température supérieure au point de fusion des cires,
(d2) en mélangeant la phase huileuse ainsi obtenue à une solution aqueuse d'un polymère cationique, et
(d3) en refroidissant l'émulsion H/E ainsi obtenue à une température inférieure au point de fusion des cires et en la mettant en contact avec la solution aqueuse d'un polymère anionique.

5. Utilisation selon au moins l'une des revendications 1 à 4,
**caractérisée en ce que**
les préparations contiennent des épaississants comme autre composant (c).

6. Utilisation selon la revendication 5,
**caractérisée en ce que**
comme composant (c) les préparations contiennent des épaississants choisis dans le groupe constitué d'acides siliciques hydrophiles, de polysaccharides, de carboxyméthylcelluloses, d'hydroxyéthylcelluloses et hydroxypropylcelluloses, de mono- et diesters d'acides gras et de polyéthylène glycol haut poids moléculaire, de polyacrylates, de polyacrylamides, d'alcools polyvinyliques, de polyvinylpyrrolidones ainsi que de bentonites.

7. Utilisation selon au moins l'une des revendications 1 à 6,
**caractérisée en ce que**
les préparations contiennent
(a) de 0,01 à 5 % en poids d'extraits végétaux,
(b) de 0,1 à 10 % en poids d'agents actifs solubles dans les graisses microencapsulés, et
(c) de 0 à 5 % en poids d'épaississants,
étant précisé que les quantités sont complétées à 100 % en poids avec de l'eau.
